# EUROPEAN PATENT APPLICATION

(11) **EP 2 009 007 A1**
(43) Date of publication of application: **31.12.2008**
(21) Application number: 07706338.6
(22) Date of filing: 15.02.2007
(51) Int. Cl.: C07D 307/86

(54) **PLANT-DERIVED THERAPEUTIC AGENT FOR MALIGNANT TUMOR**

(30) Priority: 02.03.2006 JP 2006056391
(71) Applicant: Nihon University, Tokyo 102-8275 (JP)
(72) Inventor: SUZUKI, Takashi c/o Nihon University, Tokyo 1028275 (JP); FUJIMOTO, Yasuo; c/o Nihon University, Tokyo, 1028275 (JP); UCHIYAMA, Taketo; c/o Nihon University, Tokyo, 1028275 (JP); TABATA, Keiichi; c/o Nihon University, Tokyo, 1028275 (JP)
(74) Representative: Blodig, Wolfgang
(86) International application number: PCT/JP2007/000090
(87) International publication number: WO 2007/105353

(57) **Abstract**

A therapeutic agent for a malignant tumor containing a compound represented by the following formula (1) or (2): 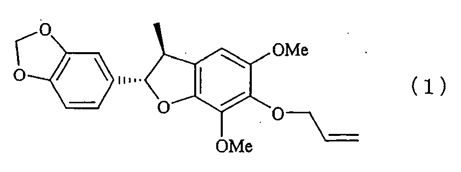 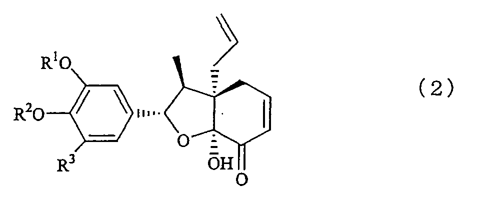 (wherein each of R¹ and R² represents a methyl group, or R¹ and R² may be linked together to form a methylene group; and R³ represents a hydrogen atom or a methoxy group).

## Description

### Technical Field

The present invention relates to a therapeutic agent for a malignant tumor which contains a plant-derived ingredient.

### Background Art

Generally, plants contain a variety of physiologically active substances, and drugs have long been produced from plant extracts. Hitherto known plant-derived therapeutic agents for a malignant tumor include Taxol, which is an extract obtained from a plant belonging to the family Taxaceae; camptothecine, which is an alkaloid contained in *Nothapodytes Foetida* (Icacinacea) or *Camptotheca Acuminata* (Nyssaceae); and vincristine and vinblastine, which are indole alkaloids contained in *Catharanthus roseus* (Apocynaceae).

Meanwhile, among neolignans, which are plant-derived ingredients, a variety of compounds are known to exhibit a pharmaceutical effect. Examples of neolignans known to exhibit cytotoxicity include Perseal F and Licarin A (Non-Patent Documents 1 and 2).
Non-Patent Document 1:
   Thai I. L., Chen J. H., Duh C. Y., Chen I.S., "Cytotoxic Neolignans and Butanolides from Machilus obovatifolia," Planta Medica 2001; 67: 559-561
Non-Patent Document 2:
   Thai I. L., Hsieh C. F., Duh C. Y., Chen I.S., "Further study on the chemical constituents and their cytotoxicity from the leaves of Persea obovatifolia," The Chinese Pharmaceutical Journal 1999; 51: 335-345

### Disclosure of the Invention

### Problems to be Solved by the Invention

An object of the present invention is to provide, through exploration of a variety of plants, a novel therapeutic agent for a malignant tumor.

### Means for Solving the Problems

The present inventors have extracted components from seeds of *Licaria puchury-major,* which is a Brazilian medicinal plant, and carried out screening of a compound of interest from extraction fractions of a variety of solvents in terms of apoptosis induction activity and anti-tumor activity as indices. As a result, the inventors have found that an acetone fraction exhibits excellent apoptosis induction activity to tumor cells. Through a study on the effective ingredient, the inventors have found that the following compounds exhibit excellent anti-tumor activity. The present invention has been accomplished on the basis of these findings.

Accordingly, the present invention provides a therapeutic agent for a malignant tumor and an apoptosis-inducing agent each containing a compound represented by the following formula (1) or (2):

### [F1]

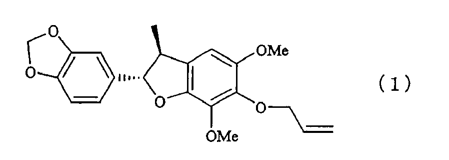

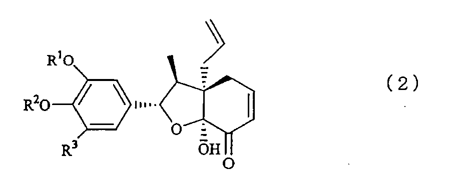

(wherein each of R¹ and R² represents a methyl group, or R¹ and R² may be linked together to form a methylene group; and R³ represents a hydrogen atom or a methoxy group).

The present invention also provides use of the compound for producing a therapeutic agent for a malignant tumor and an apoptosis-inducing agent.
The present invention further provides a method for the treatment of a malignant tumor and for inducing apoptosis, comprising administering, to a subject in need thereof, the compound in an effective amount.

### Effects of the Invention

The therapeutic agent for a malignant tumor according to the present invention exhibits excellent anti-tumor activity, which is considered to be attributed to inducing apoptosis in cancer cells. The agent of the invention is a new, useful therapeutic agent for a malignant tumor.

### Brief Description of the Drawings

Fig. 1 shows apoptosis induction activity of acetone fractions to Jurkat cells analyzed through flow cytometry;
Fig. 2 is a graph showing cytotoxicity of compound (1) to IMR-32;
Fig. 3 is a graph showing cytotoxicity of compound (2a) to IMR-32;
Fig. 4 is a graph showing cytotoxicity of compound (2b) to IMR-32;
Fig. 5 is a graph showing cytotoxicity of compound (2c) to IMR-32;
Fig. 6 is a graph showing cytotoxicity of compound (1) to SK-N-SH;
Fig. 7 is a graph showing cytotoxicity of compound (2a) to SK-N-SH;
Fig. 8 is a graph showing cytotoxicity of compound (2b) to SK-N-SH;
Fig. 9 is a graph showing cytotoxicity of compound (2c) to SK-N-SH;
Fig. 10 is a graph showing cytotoxicity of compound (1) to NB-39;
Fig. 11 is a graph showing cytotoxicity of compound (2a) to NB-39;
Fig. 12 is a graph showing cytotoxicity of compound (2b) to NB-39; and
Fig. 13 is a graph showing cytotoxicity of compound (2c) to NB-39.

### Best Modes for Carrying Out the Invention

The therapeutic agent for a malignant tumor and the apoptosis-inducing agent according to the present invention (hereinafter collectively referred to as "a therapeutic agent for a malignant tumor and the like") contain a compound represented by formula (1) or (2) as an effective ingredient. Specific examples of the compound (2) include the compounds represented by the following formulas (2a) to (2c).

### [F2]

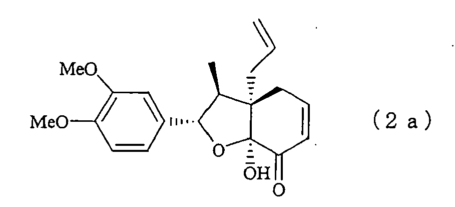

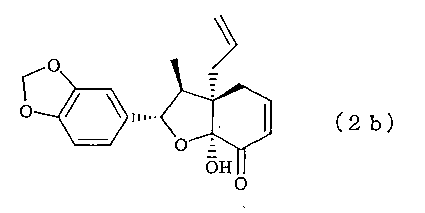

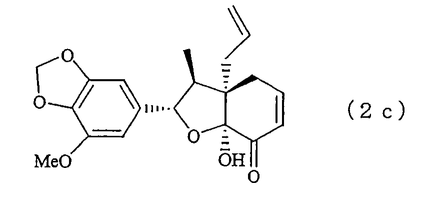

The aforementioned compounds (1) and (2) may be produced through extraction from seeds of *Licaria puchury-major,* which is a Brazilian medicinal plant. In Brazil, *Licaria puchury-major* is employed as apozem for dysentery, diarrhea, incontinence, stomach diseases, and leukorrhea, and as cataplasms and bath agents for the treatment of an injury inflicted by a poisonous insect and rheumatism (Goro HASHIMOTO, Encyclopedia of Brazilian medicinal plants).

In one procedure of extraction of the aforementioned compounds from seeds of *Licaria puchury-major,* the seeds are pulverized and subjected to extraction with ethanol. When the obtained extract is subjected to fractional extraction by use of n-hexane-90% methanol, these compounds are included in a 90% methanol fraction. The 90% methanol fraction is caused to be adsorbed by a synthetic adsorbent, and the adsorbed species are sequentially eluted with 40% methanol, 70% methanol, 100% methanol, and acetone. In the elution procedure, the compounds are mainly included in the 100% methanol fraction and the acetone fraction, particularly in the acetone fraction. Thus, the 100% methanol fraction and the acetone fraction *per se* may be employed as the therapeutic agent of the present invention for a malignant tumor and the like. For further purification, the 100% methanol fraction or the acetone fraction is purified through column chromatography.

Examples of the synthetic adsorbent include a styrene-divinylbenzene copolymer and a bromostyrene-divinylbenzene copolymer. Of these, a styrene-divinylbenzene copolymer is preferred. Examples of commercial products of the synthetic adsorbent include Daiaion HP20, Diaion HP21, Sepabeads SP825, and Sepabeads SP850.

As is clear from Examples hereinbelow, the compounds (1) and (2) exhibit excellent cytotoxicity to leukemia cells, neuroblastoma cells, etc. The cytotoxicity is attributed to apoptosis induction activity. Therefore, the compound represented by formula (1) or (2) or a *Licaria puchury-major* extract containing any of the compounds is a useful therapeutic agent for a malignant tumor of a human and other mammals.
Examples of the malignant tumor to which the therapeutic agent of the present invention is applied include tumors and solid tumors of blood and hematopoietic tissue (e.g., leukemia and lymphoma). Examples of the solid tumors include epithelial cell cancers such as skin cancer, lung cancer, colon cancer, stomach cancer, breast cancer, prostatic cancer, and thyroid gland cancer; and sarcomas such as leiomyosarcoma and osteosarcoma.

The drug of the present invention may be produced in the following procedure. Specifically, to the compound represented by formula (1) or (2) or the aforementioned extract, one or more pharmaceutically acceptable carriers including vehicles, binders, lubricants, disintegrants, coating agents, emulsifying agents, suspending agents, solvents, stabilizers, sorbents, and ointment bases are added in accordance with needs. The thus-formed mixtures are processed through a known technique to form drug preparations (pharmaceutical compositions) suitable for oral administration, injection, intrarectal administration, external application, etc.
Examples of preferred oral preparations include granules, tablets, sugar-coated tablets, capsules, pills, liquids, emulsions, and suspensions. Examples of preferred preparations for injection include such products for intravenous injection, intramuscular injection, subcutaneous injection, or drip infusion. Examples of preferred preparations for intrarectal administration include soft capsule type suppositories.
The drug of the present invention may be administered, in the form of any of the aforementioned drug products, to a human and to other mammals.
The daily dose of the drug of the present invention is preferably about 1 to 500 mg/kg as the compound (1) or (2), in a single administration or in 2-4-divided administrations.

### Examples

The present invention will next be described in more detail by way of examples, which should not be construed as limiting the invention thereto.

### Example 1

Seeds of *Licaria puchury-major* were pulverized (product: 2.8 kg). The pulverized product was subjected to extraction with ethanol (2.5 L × 3) under ultrasonication. After filtration, the ethanol extract was condensed, to thereby yield a product (dry solid content: 276.8 g). Subsequently, the condensed ethanol extract was subjected to fractional extraction by use of n-hexane-90% methanol, and the 90% -methanol fraction was condensed, to thereby yield a product (dry solid content: 93.8 g).
The 90% methanol extract was applied to a column filled with Diaion HP20 for adsorption. The adsorbed matter was sequentially eluted with 40% methanol (4 L), 70% methanol (4 L), 100% methanol (4 L), and acetone (5 L). The acetone fraction (6.95 g) was purified through silica gel column chromatography (n-hexane-ethyl acetate) and reverse phase HPLC (water-methanol), to thereby isolate compound (1) (31.4 mg), compound (2a) (187.8 mg), compound (2b) (71.7 mg), and compound (2c) (7.5 mg). The structure of these compounds were determined through ¹H-NMR, ¹³C-NMR, and EIMS. Note that compound (1), compound (2a), compound (2b), and compound (2c) are known as SN-028, SN-020, SN-017 (ferrearin C), and SN-047 (ferrearin G).

### Example 2

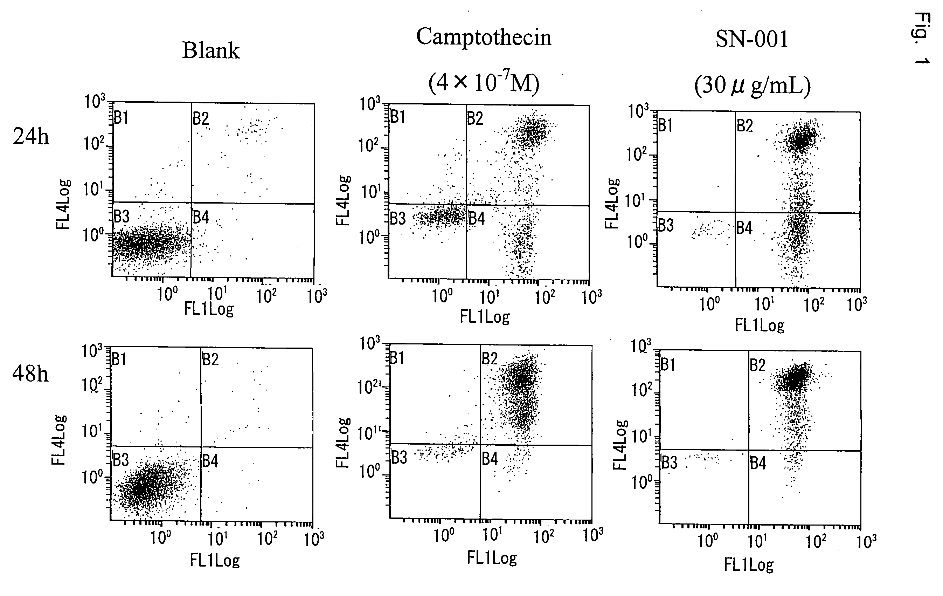

Apoptosis induction activity of fractions obtained through extraction with solvents in Example 1 to Jurkat cells, which are myelocytic leukemia cells, was determined through flow cytometry. Specifically, each extraction fraction (30 µg/mL) or camptothecine (positive control) (4 × 10⁻⁷ M) was added to Jurkat cells (5 × 10⁵ cells/2 mL-well), followed by incubation under 5% CO₂ at 37°C for 24 hours or 48 hours. The incubated cells were washed with PBS, and annexin V-FITC and propidium iodide (PI) were added to each well for flow cytometry. Through this flow cytometry, the cells at an initial stage of apoptosis emit fluorescence attributed to annexin V-FITC, whereas the cells at a later stage of apoptosis emit fluorescence attributed to both annexin V and PI.
Actually, the first ethanol extract and the methanol extraction fraction exhibited apoptosis induction activity at a high concentration. The n-hexane fraction exhibited no apoptosis induction activity. Among fractions eluted through the synthetic adsorbent, the 70% methanol fraction, the 100% methanol fraction, and the acetone fraction exhibited intense apoptosis induction activity. Fig. 1 shows results of apoptosis induction by the acetone fraction (SN-001).
As is clear from Fig. 1, the acetone fraction (SN-001) induced apoptosis to a later stage after incubation for 24 hours. This induction is earlier as compared with camptothecine.

### Example 3

The anti-malignant-tumor effect of compounds (1), (2a), (2b), and (2c) on neuroblastoma cell strains IMR-32, SK-N-SH, and NB-39 was investigated using the MTT assay.
Specifically, each type of cells was seeded onto a 96-well plate so that the cell density was adjusted to 1 × 10⁴ cells/well, and each drug compound was added to the wells 24 hours after seeding, followed by incubation under 5%-CO₂ at 37°C for 48 hours. Subsequently, an MTT solution was added to the wells, and blue formazan formed three hours after addition was well-mixed through pipetting. The absorbance of each well was measured at 570 nm (top) and 655 nm (bottom), and the survival cell rate with respect to the control group was calculated. In this assay, formation of blue formazan caused by activity of mitochondria is observed only when cells survive.

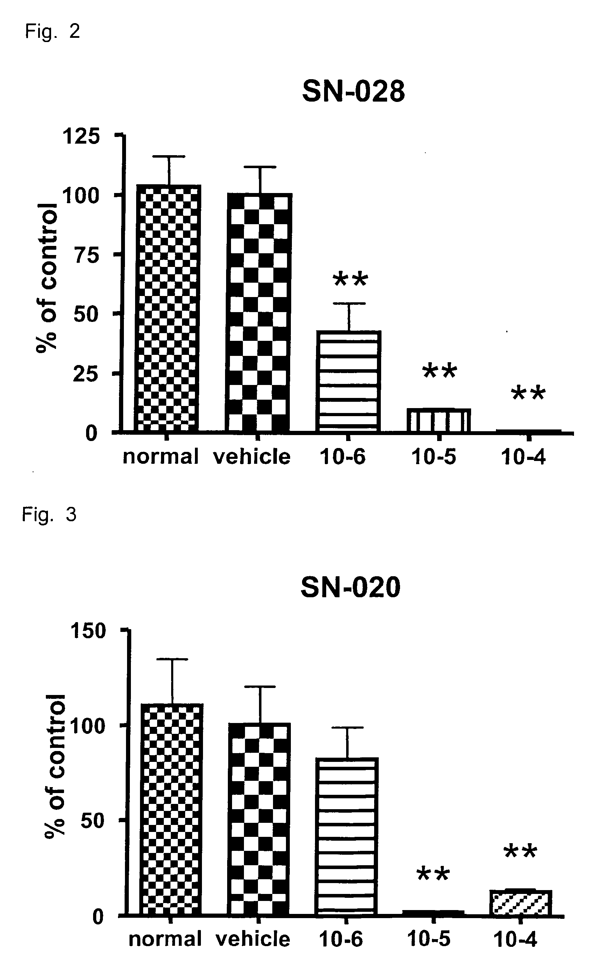

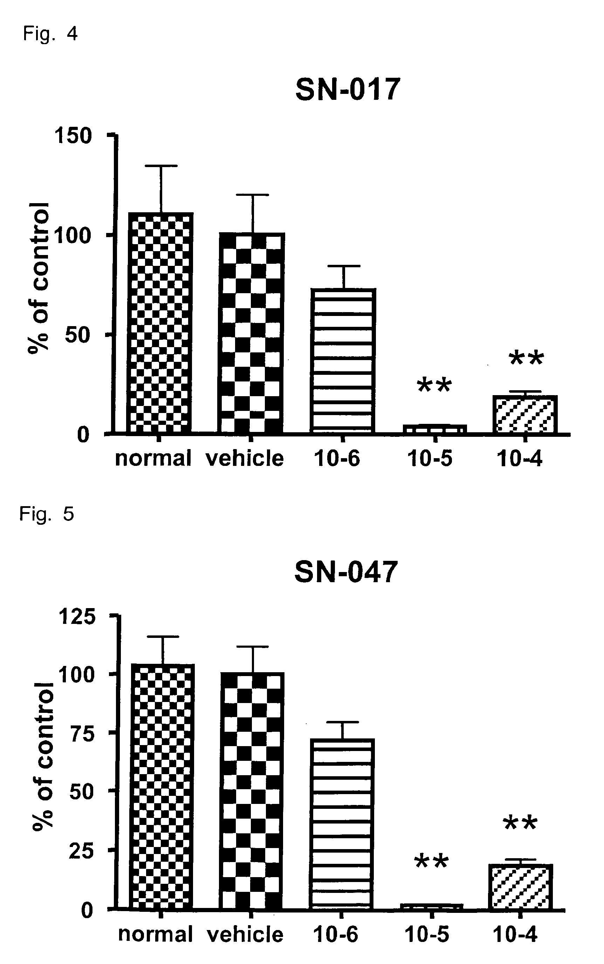

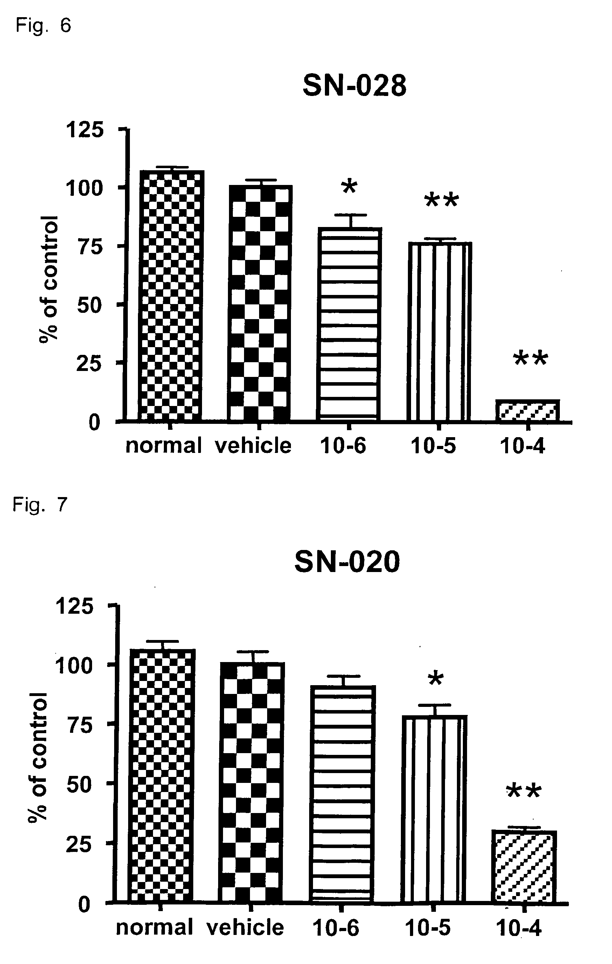

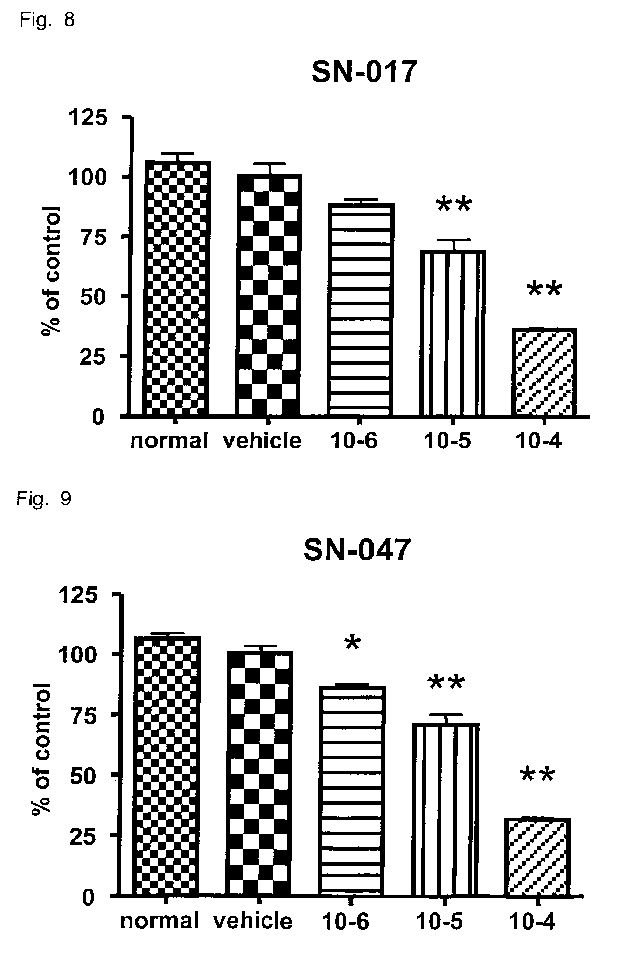

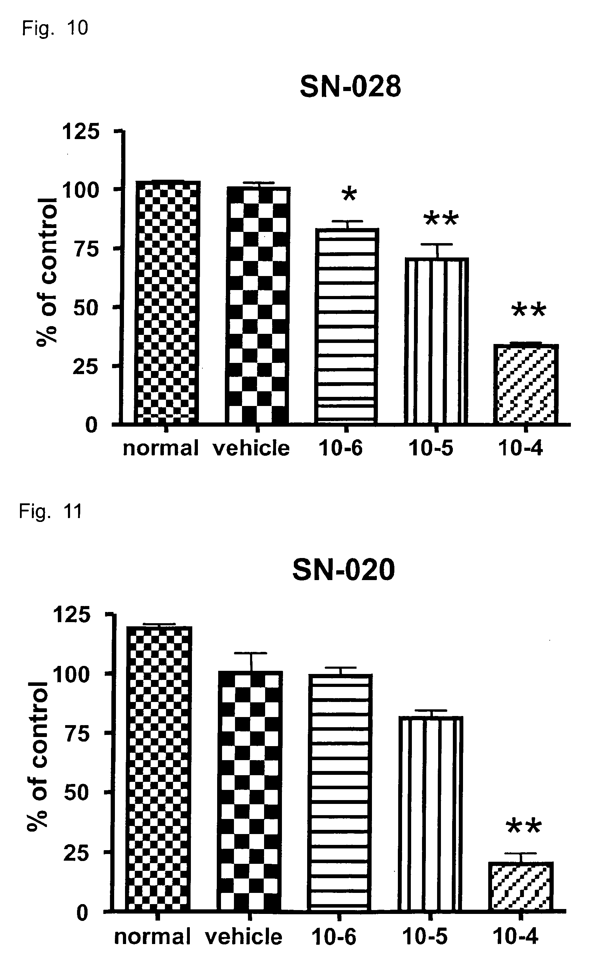

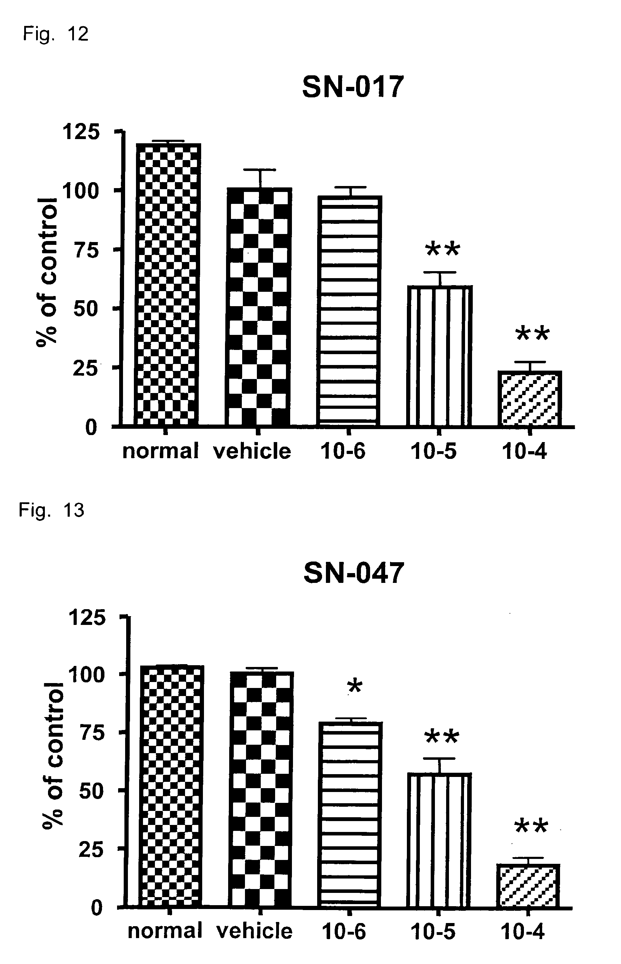

Figs. 2 to 13 shows the results. As is clear from Figs. 2 to 13, compound (1) (SN-028), compound (2a) (SN-020), compound (2b) (SN-017), and compound (2c) (SN-047) exhibited intense cytotoxicity to neuroblastoma cells.

## Claims

1. A therapeutic agent for a malignant tumor comprising a compound represented by the following formula (1) or (2):
[F1] 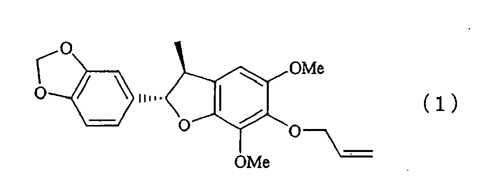 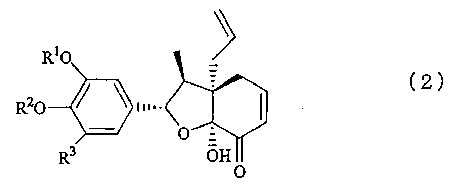 (wherein each of R¹ and R² represents a methyl group, or R¹ and R² may be linked together to form a methylene group; and R³ represents a hydrogen atom or a methoxy group).

2. An apoptosis-inducing agent comprising a compound represented by the following formula (1) or (2):
[F2] 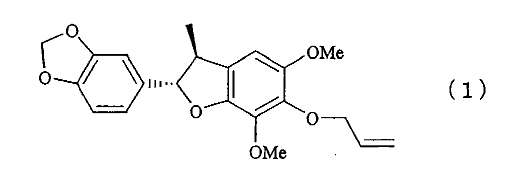 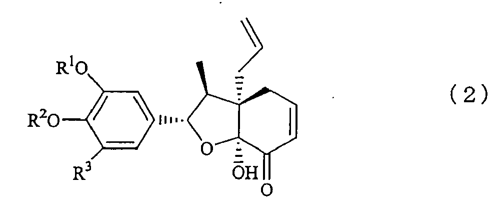 (wherein each of R¹ and R² represents a methyl group, or R¹ and R² may be linked together to form a methylene group; and R³ represents a hydrogen atom or a methoxy group).

3. Use of a compound represented by the following formula (1) or (2):
[F3] 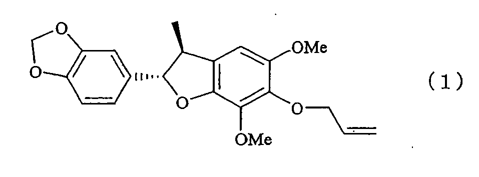 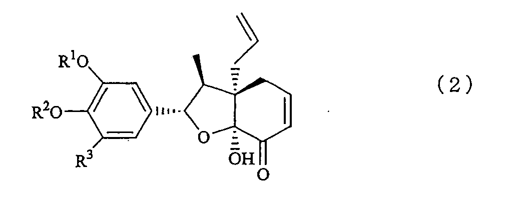 (wherein each of R¹ and R² represents a methyl group, or R¹ and R² may be linked together to form a methylene group; and R³ represents a hydrogen atom or a methoxy group) for producing a therapeutic agent for a malignant tumor.

4. Use of a compound represented by the following formula (1) or (2) :
[F4] 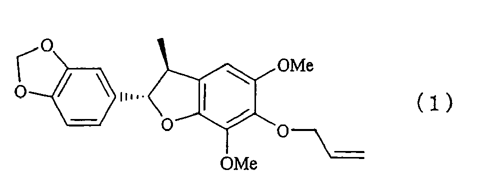 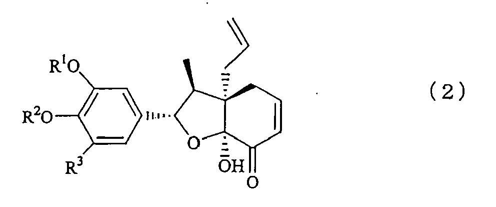 (wherein each of R¹ and R² represents a methyl group, or R¹ and R² may be linked together to form a methylene group; and R³ represents a hydrogen atom or a methoxy group) for producing an apoptosis-inducing agent.

5. A method for treatment of a malignant tumor, comprising administering an effective amount of a compound represented by the following formula (1) or (2):
[F5] 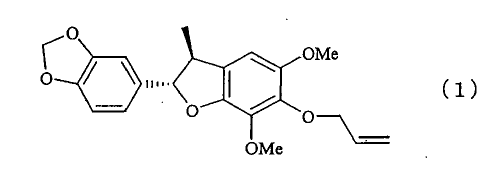 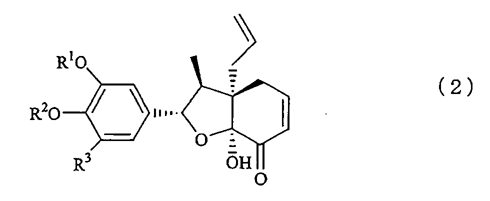 (wherein each of R¹ and R² represents a methyl group, or R¹ and R² may be linked together to form a methylene group; and R³ represents a hydrogen atom or a methoxy group) to a subject in need thereof.

6. A method for inducing apoptosis, comprising administering an effective amount of a compound represented by the following formula (1) or (2):
[F6] 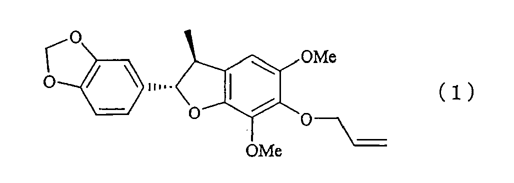 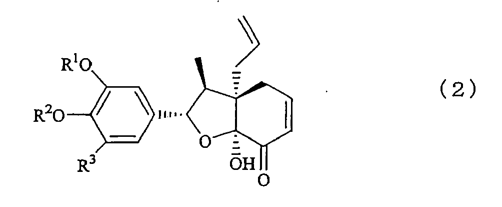 (wherein each of R¹ and R² represents a methyl group, or R¹ and R² may be linked together to form a methylene group; and R³ represents a hydrogen atom or a methoxy group) to a subject in need thereof.
